# EUROPÄISCHE PATENTSCHRIFT

(11) **EP 0 934 036 B1**
(45) Veröffentlichungstag und Bekanntmachung des Hinweises auf die Patenterteilung: **03.03.2004**
(21) Anmeldenummer: 97947724.7
(22) Anmeldetag: 28.10.1997
(51) Int. Cl.: A61F 2/06

(54) **STENT**
STENT
ENDOPROTHESE VASCULAIRE

(30) Priorität: 28.10.1996 DE 19645293; 10.12.1996 DE 19653721
(43) Veröffentlichungstag der Anmeldung: 11.08.1999
(73) Patentinhaber: BIOTRONIK Mess- und Therapiegeräte GmbH & Co Ingenieurbüro Berlin, 12359 Berlin (DE)
(72) Erfinder: KRANZ, Curt, D-10825 Berlin (DE)
(74) Vertreter: Eisenführ, Speiser & Partner
(86) Internationale Anmeldenummer: PCT/DE1997/002577
(87) Internationale Veröffentlichungsnummer: WO 1998/018407

(56) Entgegenhaltungen:
- EP-A- 0 734 698
- WO-A-96/29028
- WO-A-97/04721

## Beschreibung

Die Erfindung betrifft einen Stent, insbesondere Koronarstent, als intraluminales Expansionselement, entsprechend der im Oberbegriff des Anspruchs 1 genannten Art.

Aus den europäischen Patentschriften EP-B1 0 364 787 und EP-B1 335 341 ist ein aufweitbares intraluminales Element mit mindestens einem dünnwandigen, rohrförmigen Teil (nachfolgend als Stent bezeichnet) bekannt. Die Mantelfläche des Stents ist durchbrochen netzförmig ausgebildet und weist dabei Ausnehmungen auf, die durch sich geradlinig in axialer und in Umfangsrichtung erstreckende stegartigen Elemente von geringer Materialstärke begrenzt sind. Die stegartigen Elemente bestehen aus der restlichen Rohrwandung, von der das Material im Bereich der Ausnehmungen entfernt wurde.

Derartige Stents werden in einem operativen Eingriff unter Einwirkung von von innen nach außen gerichteten Kräften durch einen mit Druckgas beaufschlagten schlauchförmigen Dilator, expandiert. Der Stent behält dabei trotz Verformung seine Rohrform bei und weitet das durch Ablagerungen verengte Gefäß auf.

Der bekannte Stent weist den Nachteil auf, daß das Expandieren aufgrund der Verformung der sich axial erstreckenden stegartiges Elemente nur in beschränktem Maße erfolgen kann, da der Formänderung der einzelnen stegartigen Elemente des Stents relativ enge Grenzen gesetzt sind. Diese Grenzen sind bedingt durch die mit der Verformung einhergehende Materialspannungen, welche - wenn die Verformung zu stark wird - zum Bruch eines oder mehreren der das Netz bildenden stegartigen Elemente können,

Aus Sicherheitsgründen muß die Verformung deshalb normalerweise weit unterhalb eines möglichen Gefahrenbereichs gehalten werden, da der Bruch eines Steges dazu führen würde, dass dessen freie Enden im Bereich der Bruchstelle in das innere des mit dem Stent versehenen Gefäßes hineinragen würde. Durch die damit verbundene Gefahr der Bildung von Restenosen würde nicht nur der Erfolg des Eingriffs selbst infrage gestellt, sondern auch das Leben des Patienten gefährdet.

Ein Stent mit relativ geschwächten Abschnitten lokal verringerten Querschnitts, welche bei Expansion des Stents als plastische Gelenke (Ftießgelenke) dienen, in denen die Verformung konzentriert ist, ist aus der WO 96/29028 bekannt.

Ausgehend von den Mängeln des Standes der Technik liegt der Erfindung die Aufgabe zugrunde, einen expandierbaren Stent der eingangs genannten Gattung anzugeben, welcher möglichst sicher - und damit auch ohne die Gefahr eines durch Spannungsüberlastung bedingten Bruchs im Bereich der stegartiges Elemente, aufweitbar ist.

Die Aufgabe wird durch die kennzeichnenden Merkmale des Anspruchs 1 gelöst.

Die Erfindung schließt die technische Lehre ein, dass bei fragilen aus geeigneten Werkstoffen gefertigten, netzartige Strukturen aufweisenden rohrförmige Elementen, welche anwendungsbedingt einer Verformung unterzogen werden, kritische Materialbelastungen oder gar Materialbrüche vermeidbar sind, wenn in solchen Bereichen, die einer erhöhten Verformung unterworfen sind, bereits konstruktiv und von vom herein die auftretenden Spannungsmaxima begrenzt werden.

Dies geschieht hier nicht nur durch die konstruktive Auslegung in Bezug auf eine möglichst große Festigkeit durch Erhöhung der Materialquerschnitte, sondern auch dadurch, daß die Form der Stege und Verbindungsbereiche im Hinblick auf die zu erwartenden Belastungen optimiert wird. Dies erfolgt in der Weise, daß auftretende lokale Maximalspannungen nicht zum Bruch führen können, sondern lediglich zu weiterer Verformung führen.

Es ist nämlich festgestellt worden, daß Brüche deswegen vorkommen, weil das verwendete Material bei auftretenden Verformungen (im Spannungs-Dehnungs-Diagramm) nicht diejenige Zone erreicht, in der ein Fließen auftritt, sich also Spannungen durch Materialverschiebungen ausgleichen können. Nach dem Fließen tritt anschließend eine Materialverfestigung ein.

Darüber hinaus schließt die erfindungsgemäße Lösung die Erkenntnis ein, daß durch Konzentration der Verformungen auf wenige Bereiche, in diesen jeweils der Elastizitätsbereich überschritten wird, so daß die bei der Aufweitung des Stents auftretenden Verformungen "bleibend" sind, d.h. nach Entfernung des zur Aufweitung des Ballonkatheters nicht wieder "zuruckfedern". Auf diese Weise bleibt kommt das erzielbare Aufweitungsvermögen bleibend der beabsichtigten Gefäßerweiterung zugute.

In vorteilhafter Weiterbildung schließt die Erfindung dann auch die Erkennntnis ein, daß auch nach dieser eingetretenen Verfestigung eine weitere Verformung erfolgen kann, wenn in dem an den Bereich der Verfestigung anschließenden Bereich der Verjüngungsbereich weiterhin ein Erreichen des Fließzustands zuläßt.

Die bei der Verformung auftretenden Beanspruchungen werden erfindungsgemäß zunächst in der Weise minimiert, daß die einzelnen stegartigen Elemente derart geformt sind, daß die Biegeverformung, der ein stegartiges Element bei der Expansion innerhalb der hohlzylindrischen Rohrform unterworfen ist, lokal einen vorgegebenen Wert nicht überschreitet.

Gemäß der Erfindung wird bevorzugt der Querschnitt eines Stegelements in der Nähe der Verbindung zu einem weiteren Stegelement derart verjüngt ausgebildet, daß dort eine Verformung bei Expansion begünstigt ist und die Verformung lokal konzentiert erfolgt.

Dabei ist der Verjüngungsbereich derart ausgestaltet, daß eine Verformung in aneinander anschließenden Bereichen nacheinander in Richtung zunehmender Entfernung von dem Ort der Verbindung mit dem anderen Stegelement ermöglicht ist, so daß auch stärkere Verformungen ohne Gefahr eines Bruches möglich sind.

Wenn der Verjüngungsbereich derart ausgebildet ist, daß der Bereich der Verformung bei Materialverfestigung nach einem Zustand des Fließens des Materials in einem Bereich, der dem Ort der Verbindung mit einem anderen Stegelement nähergelegen ist, sich in einen benachbarten Bereich verlagert, von dem Ort der Verbindung weiter entfernt ist, wandert der Fließbereich auf dem stegartigen Element während der Verformung von einer Position in der Nähe des Verbindungsbereichs mit einem weiteren Stent zu einer davon entfernter gelegenen.

Die Dimensionierung erfolgt dabei in der Weise, daß der Widerstand gegen Verformung durch Biegung im Schwächungsbereich in Richtung zunehmender Entfernung von dem Ort der Verbindung mit dem weiteren Stegelement auch unter Berücksichtigung des sich mit der Verlagerung des Biegebereichs verkürzenden Hebelarms der an dem von dem Verbindungsbereich entfernten Bereich des Stegelements angreifenden Kräfte zunimmt.

Dabei ist der Verjüngungsbereich derart auszubilden, daß der Widerstand gegen Verformung weniger stark zunimmt, als der sich nach dem durch Materialverformung eintretenden Fließzustand erhöhenden Materialverfestigung. Dies kann durch gleichbleibende Querschnittsabmessungen des verformten Stegbereichs, gegebenenfalls aber auch im Rahmen einer Querschnittsänderung erfolgen. Hierbei muß der sich ändernde Hebelarm der angreifenden Verformungskraft durch Wandern des Fließbereichs berücksichtigt werden.

Bei einer bevorzugten weiteren Ausgestaltung der Erfindung sind in der Nähe der Schwächungsbereiche noch zusätzliche Anschlage vorgesehen, welche die Verformung in den Schwachugnsbereichen begrenzen. Die Anschläge sind dabei so vorgesehen, daß sie bei expandierendem Stent in die Nähe von benachbarten Anschlägen geraten und sich an diesen abstützen. Auf diese Weise ist eine weitere Verformung des bisherigen Schwächungsbereichs verhindert und die Verformung setzt sich in einem anderen Bereich fort. Damit sind dann lokale Materialüberbelastungen verhindert, ohne daß die Verformbarkeit des Stents eingeschränkt ist. Durch die Anschläge läßt sich die lokale Verformung begrenzen, so daß auch die Gefahr eines Bruches vermindert ist.

Mit der Erfindung wird erreicht, daß die Verformungen sich kontrolliert lokal konzentrieren, so daß die Expansion des Stents weitgehend auf eine plastische Verformung reduziert wird. Damit läßt der sich sehr fein in seiner Ausdehnung kontrollieren, ohne daß Überdehnungen auftreten, welche sich auch nachteilig auf die Gefäßwandung auswirken können.

Die Verjüngungen und Anschlage werden bevorzugt durch Variation der Stegbreite bei konstanter Materialstärke in tangentialer Richtung gebildet. Dies kommt der Erzeugung des Stents aus einem Rohr durch Ausschneiden der Aussparungen mittels eines Laser-Schneidgeräts entgegen. Zusätzlich oder alternativ dazu kann die Materialschwächung auch durch Abtrag von Material in radialer Richtung erzeugt werden. Dies kann ebenfalls mittes Laserstrahlung oder aber auch durch chemische Mittel durch Anätzen oder dergl. erfolgen. Es ist ersichtlich, daß Schwächungen aber auch auf andere Art und Weise erzeugt werden können. So können beispielsweise die geschwächten Bereiche dadurch erzeugt werden, daß nur diese nicht verstärkt - also nicht einer zusätzlichen materialauftragenden Behandlung - wie Beschichten, Plattieren, Sputtern etc. unterworfen werden.

Nach einer anderen gunstigen Ausführungsform der Erfindung weisen die Koppelglieder im wesentlichen dieselbe(n) Form(en) auf wie die Koppelglieder zwischen den benachbarten expandierbaren Elementen der einzelnen Segmente des Stents, so daß sich auch an diesen Verbindungspunkten keine Kerbspannungsspitzen beim Expandieren des Stents ausbilden können. Auch derartige Koppelglieder weisen Verjüngungen nach den hier beschriebenen Gesichtspunkten auf.

Eine bevorzugte Stentausführung gemäß der vorstehenden Auslegung besteht aus Tantal als Werkstoff und ist mit einer Beschichtung aus amorphem Siliciumcarbid versehen.

Weitere Einzelheiten von derartigen Stents ergeben sich aus einer Anzahl von gleichzeitig eingereichten Patentanmeldungen derselben Anmelderin.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine bevorzugte Ausführungsform der Erfindung in Seitenansicht,
Figuren 2 a bis c ein Detail der in Figur 1 dargestellten Ausführungsform in verschiedenen Stufen beim Expandieren des Stents,
Figur 2d das Spannungs-Dehnungs-Diagramm von Rein-Titan,
Figur 3 ein weiteres Ausführungsbeispiel eines erfindungsgemäßen Stents in der Abwicklung,
Figuren 3a und 3b Details von Figur 3,
Figur 4a das Ausführungsbeispiel gemäß Figur 3 in teilweise expandiertem Zustand sowie
Figur 4b das Ausführungsbeispiel gemäß Figur 3 in vollständig expandiertem Zustand.

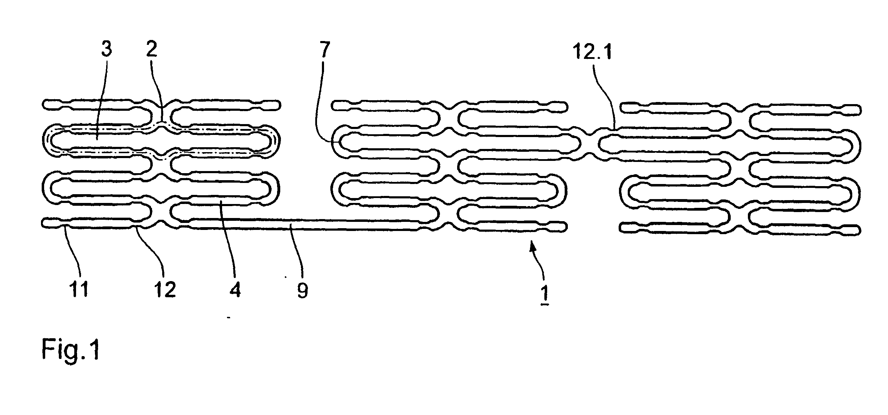

Der in Figur 1 wiedergegebene Stent 1 weist eine rohrförmig/hohlzylindrische Grundform mit zahlreichen Durchbrüchen auf, welche von expansiblen Strukturelementen umschlossen sind, die die Form von gestauchten Ringen aufweisen. Diese werden nachfolgend als "expansible Elemente" bezeichnet und sind an einem Beispiel mit einer strichpunktierten Linie 2 markiert. Diese expansiblen Elemente 2 werden gebildet durch umlaufende schmale stegartige Bereiche 4 mit rechtekkigem Querschnitt und zeichnen sich dadurch aus, daß sie eine Ausnehmung 3 ringförmig umschließen.

Bei dem dargestellten Ausführungsbeispiel ist der Querschnitt eines der Stegelemente 4 in der Nahe der Verbindung zu einem weiteren Stegelement derart verjüngt, daß dort eine Verformung bei Expansion begünstigt ist. Diese Verjüngungsbereiche sind beispielsweise mit 11 und 12 bezeichnet.

Der Verjüngungsbereich ist derart ausgestaltet, daß eine Verformung in lokal aneinander anschließenden Bereichen nacheinander in Richtung zunehmender Entfernung von dem Ort der Verbindung mit dem anderen Stegelement ermöglicht ist.

Die expansiblen Elemente 2 haben in vollständig expandiertem Zustand (in der Zeichnung nicht dargestellt) nahezu die Form eines Vielecks. Die expansiblen Elemente sind derart geformt, daß sie sich nach dem Einbringen des Stents in ein Gefäß durch Dilatieren mit einem Ballonkatheter mit minimaler Verformung in die Polygonalform umwandeln.

Die in Figur 1 dargestellte Ausführung eines Stents ist in mehrere, in axialer Richtung aufeinanderfolgender Segmente untergliedert. Diese Segmente sind unter sich gleichartig ausgebildet und weisen eine Mantelfläche auf, in welcher Ausnehmungen 3 in tangentialer Richtung aneinandergereiht und durch Verbindungsbereiche miteinander verbunden sind.

Zur Verbindung zwischen den benachbarten Segmenten 2 sind Stegbereiche vorgesehen, welche ebenfalls Verjüngungen aufweisen. Dies ist am Beispiel des Verjüngungsbereichs 12.1 dargestellt. Diese Koppelelemente gestatten es den Segmenten, sich noch stärker an Krümmungen oder Verzweigungen von Gefäßen anzupassen.

Die vorstehend beschriebene Ausbildung der Stents gestattet ein Expandieren der rohrförmigen Stents, ohne daß es an den Verbindungspunkten zur Ausbildung von zur Zerstörung von Stegbereichen führenden Extremwerten der Kerbspannung kommt.

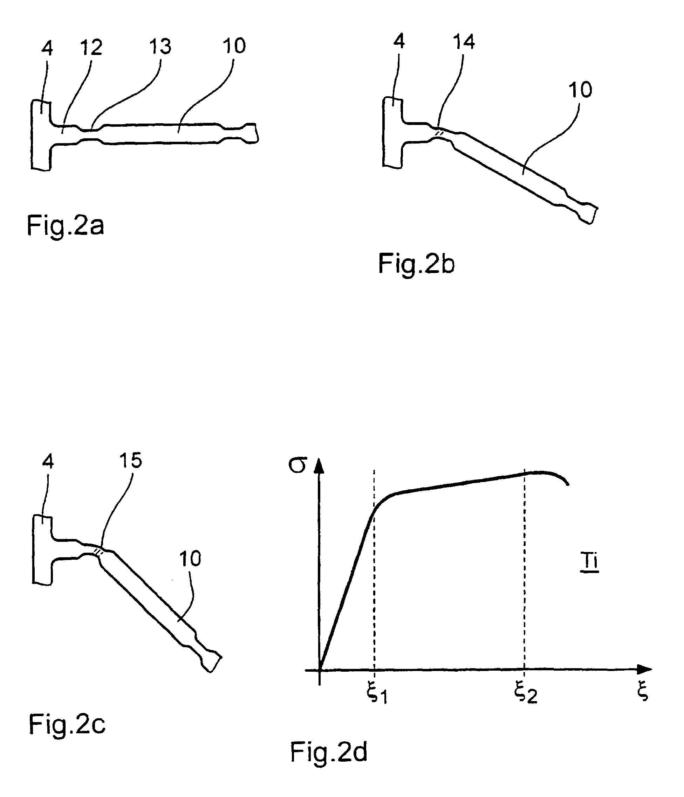

In den Figuren 2a bis c ist dargestellt, wie mit zunehmender Verformung eines stegartigen Bereichs 10, der an einen weiteren stegartigen Bereich angrenzt, bei dem es sich auch - wie bei der in Figur 1 dargestellten Ausführung - um einen Kreuzungs- oder Krümmungsbereich handeln kann, ausgehend von dem gestreckten Zustand (Figur 2a) eine Verformung zunächst in dem der Verbindung mit einem weiteren Stegbereich benachbarten Teil 14 des Verjüngungsbereichs 13 eintritt. Der Fließbereich ist hier schraffiert dargestellt. Nach Verfestigung des ursprünglichen Fließbereichs 14 wandert der Fließbereich dann mit zunehmender Verformung in einen vom Verbindungsbeich weiter entfernt gelegenen Bereich 15.

Es ist ersichtlich, daß hier der Verjüngungsbereich derart ausgestaltet ist, daß der Bereich der Verformung bei Materialverfestigung nach einem Zustand des Fließens des Materials in einem Bereich, der dem Ort der Verbindung mit einem anderen Stegelement nahergelegen ist, sich in einen benachbarten Bereich verlagert, der von dem Ort der Verbindung weiter entfernt ist. Der Widerstand gegen Verformung durch Biegung im Schwächungsbereich nimmt in Richtung zunehmender Entfernung von dem Ort der Verbindung mit dem weiteren Stegelement auch unter Berücksichtigung des sich mit der Verlagerung des Biegebereichs verkürzenden Hebelarms der an dem von dem Verbindungsbereich entfernten Bereich des Stegelements angreifenden Kräfte zu. Der Widerstand gegen Verformung nimmt hierbei weniger stark zu, als der sich nach dem durch Materialverformung eintretenden Fließzustand erhöhenden Materialverfestigung. Der Verjüngungsbereich ist dabei lediglich durch Variation der Stegbreite bei konstanter Materialstärke in tangentialer Richtung gebildet.

Die Expansion kann damit ohne lokale Überdehnungen ausgeführt werden, welche die Gefahr eines Bruches einschließen könne Zur Verdeutlichung der vorstehend dargestellten Zusammenhänge ist in Figur 2d das Spannungs-Dehnungs-Diagramm (am Beispiel von Rein-Titan) dargestellt. Es ist ersichtlich, daß anschließend an einen elastischen Bereich mit linearem Anstieg der Spannung ε zwischen 0 und ε₁, der einem "federnden" Verhalten entspricht, bis ε₂ ein plastischer Fließbereich anschließt. In diesem Bereich findet eine Materialumformung ohne Rückfederung statt, so daß der Stent im wesentlichen diejenige expandierte Form vollständig beibehält, in die er durch den Dilations-Katheter gebracht worden ist. Hierbei sollte der obere Spannungsbereich ε₂ nicht überschritten werden, da die Gefahr eines Bruches bei weiterer Spannungserhöhung in Betracht zu ziehen ist. Diese Gefahr wird durch die bei dem nachfolgend beschriebenen Ausführungsbeispiel zusätzlich vorgesehenen Anschlage ausgeschlossen.

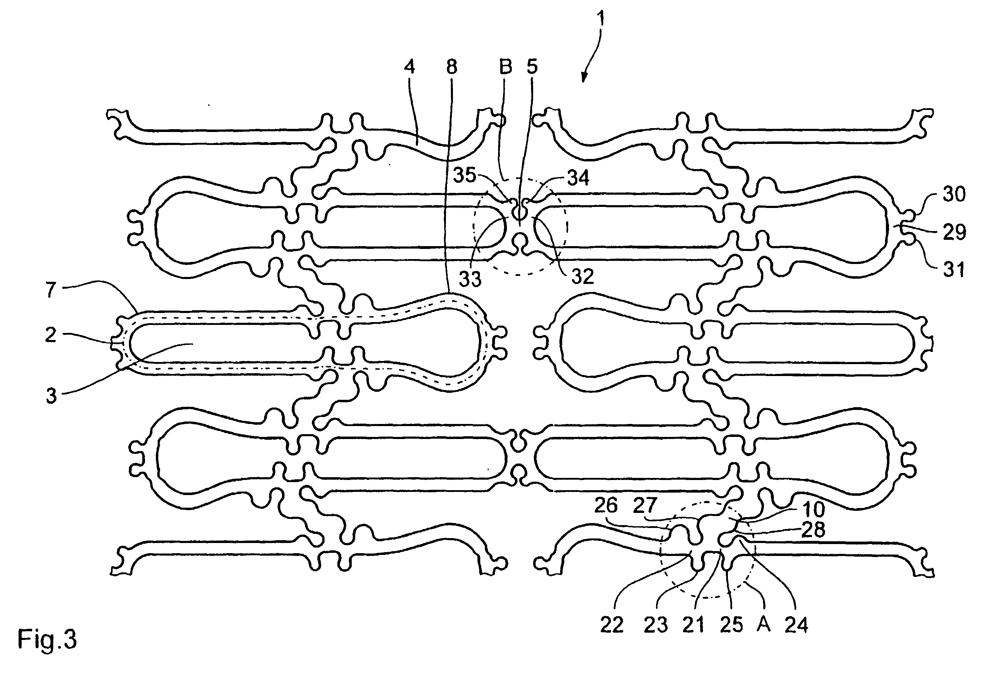

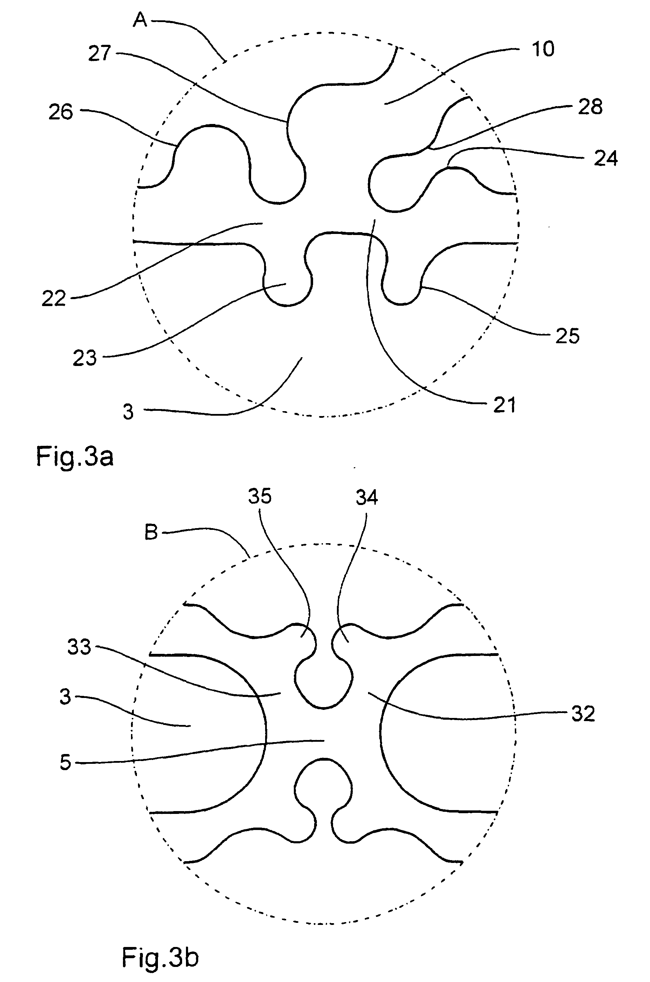

Auch ein in Figur 3 als weiteres Ausführungsbeispiel wiedergegebener Stent 1 weist eine rohrförmig/hohlzylindrische Grundform mit zahlreichen Durchbrüchen auf, welche von expansiblen Strukturelementen umschlossen sind, die in diesem Fall die Form von gestauchten Ringen aufweisen. (Die Bezugszeichen werden entsprechend dem zuvor dargestellten Ausführungsbeispiel verwendet.) Entsprechend werden auch hier expansible Elemente 2 gebildet durch umlaufende schmale stegartige Bereiche 4 mit rechteckigem Querschnitt und zeichnen sich dadurch aus, daß sie eine Ausnehmung 3 ringförmig umschließen. Die expansiblen Elemente 2 haben in diesem Fall in vollständig expandiertem Zustand nahezu die Form eines Kreises oder einer Ellipse. Es ist ersichtlich, daß die Form im nicht expandierten Zustand aus der Form des expandierten Zustands abgeleitet ist, obwohl der Stent, wenn er hergestellt ist, diesen Zustand nie eingenommen hat. Die Form wurde gefunden, indem in einem simulierten Verfahren - ausgehend einer im expandierten Zustand einzunehmenden Idealform - die Kompression in einer Modellrechnung simuliert wurde.

Die ein expansibles Element 2 umschließenden stegartigen Bereiche 4 sind dabei mehrfach S-förmig geschwungen ausgebildet. Sie umschließen jeweils eine Ausnehmung 3 in der Weise, daß die sich in tangentialer Richtung benachbart gegenüberliegenden stegartiges Bereiche 4 der selben oder einer benachbarten Ausnehmung 3 spiegelsymmetrisch angeordnet sind. An ihren in Längsrichtung gelegenen Enden weisen die expansiblen Elemente 2 freie Bogenbereiche 7 und 8 mit vergrößerter Krümmung auf.

Die expansiblen Elemente 2 sind derart geformt, daß sie sich nach dem Einbringen des Stents in ein Gefäß durch Dilatieren mit einem Ballonkatheter nahezu in eine Ringform umwandeln. Es ist ersichtlich, daß dabei der Bogen 8 einen maximalen Radius einnimmt. Durch die S-förmigen Gegenbögen ist es ihm ermöglicht, bei der Expansion eine möglichst geringe Verformung zu erleiden.

Zwischen den in axialer (Längs-) und in tangentialer (Quer) Richtung benachbart auf der Mantelfläche des Stents 1 angeordneten Ausnehmungen 3 sind Verbindungsbereiche 5 vorgesehen, welche die jeweiligen expandierbaren Bereiche 2 mechanisch miteinander koppeln. Im kreuzenden Verbindungsbereich 5 sind dabei Materialverrundungen derart vorgesehen, so daß der Verbindungsbereich eine organische, das Auftreten von erhöhten Kerbspannungen vermindernde Form aufweist.

Die Form des Stents entspricht in nicht expandiertem Zustand im wesentlichen derjenigen Form, die sich ergibt, wenn ein in expandierter Form Stegstrukturen von regelmäßiger Form aufweisendes, in dieser Form aus einer rohrförmigen Struktur erstelltes Muster, in die nicht expandierte Form - die spätere Ausgangsform - komprimiert wird. Die regelmäßige Form besteht aus Kreisen, Ellipsen, Rechtecken, Quadraten, Vielecken oder aus diesen zusammengesetzten bzw. an diese angenäherten Gebilden.

Verzweigungen von Stegen sind unter Vermeidung von sprungartigen Änderungen der Stegbreite derart gestaltet, daß die Materialspannungen insbesondere im Bereich der Verzweigung bei der Verformung auch als Kerbspannung einen vorgegebenen Wert nicht überschreitet. Der in Figur 1a wiedergegebene Stent ist über seine gesamte Länge im wesentlichen homogen strukturiert.

Es ist ersichtlich, daß bei der dargestellten Ausführungsform in Längsrichtung des Stents jeweils zwei in Querrichtung expansible Elemente 2 stirnseitig unmittelbar miteinander verbunden sind, wobei jedes dieser beiden expansiblen Elemente, in Form eines aus Stegen gebildeten gestauchten Ringelements, in Querrichtung über je ein stegartiges Element mit jeweils einem weiteren in Querrichtung expansiblen Element verbunden ist, welches seinerseits nicht mit einem anderen expansiblen Element stirnseitig unmittelbar verbunden ist, das wiederum mit einem der erstgenannten in Querrichtung expansiblen Elemente mit einem stegartiges Element verbunden ist, wobei die stegartigen Elemente jeweils in einem solchen Winkel zur Querrichtung geneigt verlaufen, daß dieser Winkel sich bei Expansion des Stents verringert und sich somit die nicht verbundenen benachbarten stirnseitigen Enden von in Querrichtung expandierbaren Elementen voneinander entfernen können auch Verkurzungen des Stents bei dessen Expansion vermieden werden.

Die stegartigen Elemente weisen bei nicht expandiertem Stent eine Neigung zur Querrichtung von im wesentlichen 45° auf.

Durch den bei der Expansion des Stents gleichzeitig auftretenden Vorgang der Dehnung der expandierbaren Elemente in Querrichung des Stents und durch das Ausrichten der schrägstehenden Verbindungselemente 10 in Querrichtung werden die jeweils nicht miteinander verbundenen Gruppen von expansiblen Elementen so gegeneinander verschoben, daß diese Bewegung die Verkürzung des Stents durch Ausdehnung der flachen Formen der expansiblen Elemente zu O-Ringen kompensiert.

In Figur 3 - und den vergrößerten Detailzeichnungen gemäß Figuren 3a und 3b - ist erkennbar, daß hier in der Nähe der Schwächungsbereiche noch zusätzliche Anschläge vorgesehen sind, welche die Verformung in den Schwächungsbereichen begrenzen. Die Anschläge sind dabei so vorgesehen, daß sie bei expandierendem Stent in die Nähe von benachbarten Anschlägen geraten und sich an diesen abstützen. Auf diese Weise ist eine weitere Verformung des bisherigen Schwächungsbereichs verhindert und die Verformung setzt sich in einem anderen Bereich fort. Damit sind dann lokale Materialüberbelastungen verhindert, ohne daß die Verformbarkeit des Stents eingeschränkt ist.

Die Wirkung der Anschläge und Schwächungen soll am Beispiel der Verzweigung im Bereich A dargestellt werden, welche in Figur 3a noch einmal vergrößert wiedergegeben ist. Das Verbidnungselement 10 zweigt von einem expansiblen Element 2 ab. Der Verbindungsbereich des Verbindungselements geht in zwei Schwächungsbereiche 21 und 22 über. Das Verbindungselement 10 ist im Übergangsbereich ebenfalls geschwächt. An die Schwächungsbereiche schließen verstärkte Anschlage 23, 24, bzw. 25 und 26 an. An dem Verbindungselement sind die Anschläge 27 und 28 vorgesehen. Es ist ersichtlich, daß bei der Expansion des Stents sich die Anschläge 23 und 25, 26 und 27 sowie 24 und 28 jeweils aneinander annähern und eine weitere Verformung des jeweils dazwischen gelegenen Schwächungsbereichs verhindern, wenn sie aneinander anliegen. Die weitere Verformung konzentriert sich dann auf die jenseits der durch die Verdickungen gebildeten Anschläge gelegenen Bereiche, welche ebenfalls eine Art "Schwächungen" bilden und dann die weiteren Verformungen aufnehmen. Auf diese Weise läßt sich die Gesamtverformung auf eine Anzahl von Verformungsbereichen "aufteilen", welche jeweils plastisch verformt werden und somit nicht "federnd" wirken.

Entsprechendes gilt für die im Verbindungsbereich 5 gelegenen Schwächungsbereiche 32 und 33 sowie die dazugehörigen Anschläge 34 und 35. Eine detaillierte Darstellung dazu ist in Figur 3b wiedergeben (Detail B in Figur 3).

Auch in den außen gelegenen Bögen der expansiblen Elemente ist ein Schwächungsbereich 29 in Verbindung mit Anschlägen 30 und 31 und lokalisiert auf die beschriebene Weise die auftretenden Verformungen.

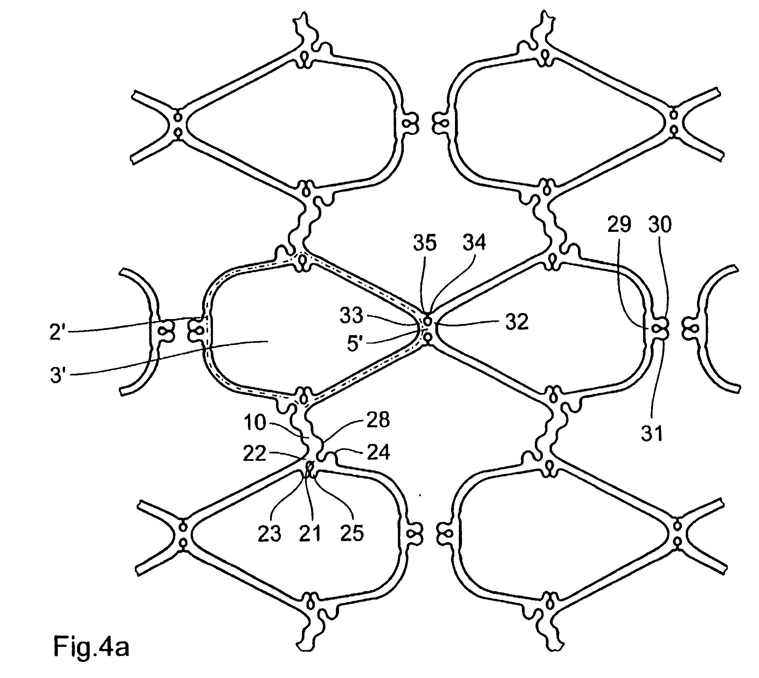

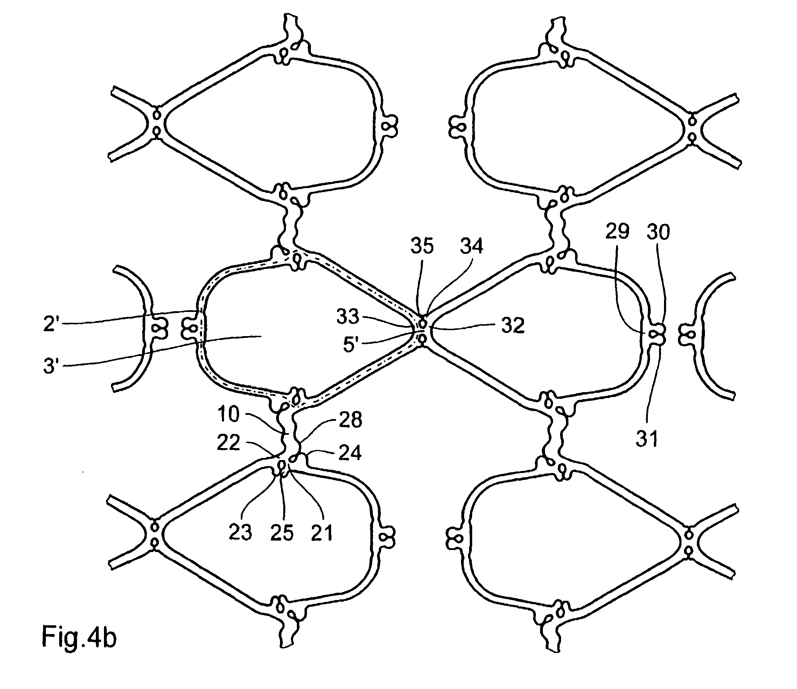

In den Figuren 4a und b ist - unter Verwendung derselben Bezugszeichen wie in Figur 3 - ersichtlich, wie die Struktur gemäß Figur sich in expandiertem Zustand zu einer Struktur mit weitgehend runden Ringen formt, die mit Stegen untereinander verbunden sind. Die Verbindungsstege zwischen den expansiblen Elementen sind jetzt weitgehend tangential ausgerichtet.

Es ist anhand der Figuren 4a und 4b ersichtlich, wie die Verformung "zweistufig" erfolgt, wobei sich zunächst beispielsweise die Anschläge 22 und 23, benachbart zu dem Schwächungsbereich 21 aneinander annähern (Figur 4a). Entsprechendes gilt für die Anschlä#ge 30 und 31 in Bezug auf die Schwächung 29 sowie für die Anschläge 34 und 35 in Bezug auf die Schwächung 33 im Bereich der Verbindungsstelle 5.

Anschließend im Verlauf der weiteren Expansion (Figur 4b) nähern sich dann auch weitere Anschläge 24 und 28 aneinander an oder es wird der Ort der Verformung in Bereiche übergeleitet, die von der Schwächung aus gesehen, jenseits der Anschläge gelegen sind.

Es ist ferner erkennbar, daß Verzweigungen von Stegen unter Vermeidung von sprungartigen Änderungen der Stegbreite ausgebildet sind, und daß Verzweigungen derart ausgebildet sind, daß die Materialspannungen insbesondere im Bereich der Verzweigung bei der Verformung auch als Kerbspannung einen vorgegebenen Wert nicht überschreitet.

Die vorstehend beschriebene Ausbildung der Stents 1 gestattet ein Expandieren der rohrförmigen Stents, ohne daß es an den Verbindungspunkten zur Ausbildung von zur Zerstörung von Stegbereichen führenden Extremwerten der Kerbspannung kommt.

Aus den Figuren 4a und b ist ferner erkennbar, daß bei der Dilatation nicht nur keine Verkürzung, sondern sogar eine gewisse Längung des Stents eintritt, die jedoch bei weiterer Expansion wieder dahingehend kompensiert wird, daß die Länge des Stents wieder der Ausgangslänge entspricht. Damit ist eine weitestgehend komplikationsfreie Anwendung gesichert. Das stegartige Verbindungselement 11' ist bei dem in dieser Figur dargestellten Ausführungsbeispiel doppelt s-förmig gekrümmt ausgebildet, um die Verformungen zu minimieren.

Aus den Figuren ist weiterhin ersichtlich, daß der erfindungsgemäße Stent auch eine sehr gute Verformbarkedit aufweist, da die Elemente 2 immer nur wechselweise miteinander verbunden sind.

Der hier dargestellte Stent besteht aus Titan oder Tantal als Werkstoff, woraus eine gute Körperverträglichkeit und eine ausgezeichnete Verformbarkeit resultiert. Auch andere biokompatible Metalle bzw. Metallegierungen sind geeignet.

Eine Mikrobeschichtung aus amorphem Siliciumcarbid wirkt einer Thrombenbildung entgegen.

Die Erfindung beschränkt sich in ihrer Ausführung nicht auf die vorstehend angegebenen bevorzugten Ausführungsbeispiele. Vielmehr ist eine Anzahl von Varianten günstig, welche von der dargestellten Lösung auch bei grundsätzlich anders gearteten Ausführungen Gebrauch macht.

## Patentansprüche

1. Stent (1), insbesondere Koronarstent, bestehend aus mindestens einem dünnwandigen, rohrförmigen Element, dessen Mantelfläche durchbrochen netzförmig ausgebildet ist und dabei Ausnehmungen (3) aufweist, die durch stegartige Elemente (4) von geringer Breite begrenzt sind, wobei die stegartigen Elemente insbesondere den Rest einer Rohrwandung bilden, aus der das Material im Bereich der Ausnehmungen entfernt wurde, wobei der Querschnitt eines Stegelements in der Nähe des Verbindungsbereichs (5) mit einem weiteren Stegelement in einem Bereich seiner Längsrichtung derart verjüngt ist, dass, wenn eine Auslenkung des stegartigen Elements durch Kraftangriff im Bereich seines vom Verbindungsbereich (11, 12) entfernten Endes erfolgt, in dem verjüngten Bereich eine über den gesamten Stegquerschnitt plastische Verformung eintritt, **dadurch gekennzeichnet, dass** Anschläge (23, 24) vorgesehen sind, um die plastische Verformung zu begrenzen, bevor eine Festigkeitsverminderung durch Rissbildung oder dergleichen eintritt, wobei die Anschläge durch Erweiterungen der Stege, außerhalb von Schwächungsbereichen, und/oder diesen bei Verformung benachbarten Bereiche gebildet werden, welche sich bei Verformung mit der Expansion des Stents aneinander annähern und eine weitere Verformung des Schwächungsbereichs (21, 22) im wesentlichen verhindern.

2. Stent nach Anspruch 1, **dadurch gekennzeichnet, dass** die Verformung unter Erreichung des Fließzustands des Materials eintritt.

3. Stent nach Anspruch 1, **dadurch gekennzeichnet, dass** der Verjüngungsbereich derart ausgestaltet ist, dass eine Verformung in lokal aneinander anschließenden Bereichen nacheinander in Richtung zunehmender Entfernung von dem Ort der Verbindung mit dem anderen Stegelement erfolgt.

4. Stent nach Anspruch 1, **dadurch gekennzeichnet, dass** der Verjüngungsbereich oder ein dem Verjüngungsbereich benachbarter Bereich derart ausgestaltet ist, dass der Bereich der Verformung bei einer nach plastischer Verformung eintretender Materialverfestigung in einem ersten Bereich, der dem Ort der Verbindung mit einem anderen Stegelement nähergelegen ist, in einen benachbarten Bereich verlagert, der von dem Ort der Verbindung weiter entfernt ist als der Bereich der Verfestigung.

5. Stent nach Anspruch 1, **dadurch gekennzeichnet, dass** der Widerstand gegen Verformung weniger stark zunimmt, als der sich nach dem durch Materialverformung eintretenden Fließzustand erhöhenden Materialverfestigung.

6. Stent nach Anspruch 1, **dadurch gekennzeichnet, dass** der Verjüngungsbereich durch Variation der Stegbreite bei konstanter Materialstärke in tangentialer Richtung und/oder durch Materialabtrag in radialer Richtung gebildet ist.

7. Stent nach Anspruch 1, **dadurch gekennzeichnet, dass** der Verjüngungsbereich in Stegbereichen vorgesehen ist, welche im nicht expandierten Zustand des Stents eine zur Längsrichtung geneigte Position innerhalb der hohlzylindrischen Oberfläche einnehmen.

8. Stent nach Anspruch 1, **dadurch gekennzeichnet, dass** die Kanten von stegartigen Elementen in einem Kreuzungs- oder Verzweigungsbereich oder im Bereich eines aufeinanderfolgende Stentsegmente verbindenden Verbindungsbereiches (5) Verrundungen aufweisen.

9. Stent nach Anspruch 1, **dadurch gekennzeichnet, dass** den Anschlägen weitere Schwächungsbereiche mechanisch nachgeschaltet sind, welche bei weiterer Expansion des Stents eine weitere Verformung ermöglichen, wenn die Verformung des ersten Schwächungsbereichs durch die Anschläge im wesentlichen verhindert ist.

10. Stent nach Anspruch 1, **dadurch gekennzeichnet, dass** weitere Anschläge vorgesehen sind, welche die Annäherung benachbarter stegartiger Elemente bei Kompression oder Krimpen begrenzen.

11. Stent nach Anspruch 1, **dadurch gekennzeichnet, dass** mehrere, in axialer Richtung reihenförmig aufeinanderfolgende Segmente (2) vorgesehen sind, die jeweils durch ein stegartiges Element verbunden sind, welches mit einem Verjüngungsbereich versehen ist.

12. Stent nach Anspruch 1, **dadurch gekennzeichnet, dass** sich die Position des Verbindungsbereichs (6, 6') zwischen mehreren Segmenten (2) von Segment zu Segment in tangentialer Richtung ändert.

13. Stent nach Anspruch 1, **dadurch gekennzeichnet, dass** außerhalb der Schwächungsbereiche Verformungsbereiche vorgesehen sind, in denen bei der Expansion des Stents höchstens Verformungen auftreten, in denen keine die Festigkeit der Struktur beeinträchtigenden Verformungen auftreten.

14. Stent nach Anspruch 1, **dadurch gekennzeichnet, dass** der Stent aus Titan, Tantal oder einen anderen biokompatibten Metall bzw. einer entsprechenden Metallegierung als Werkstoff besteht.

15. Stent nach Anspruch 1, **dadurch gekennzeichnet, dass** eine Beschichtung aus amorphem Siliciumcarbid vorgesehen ist.

## Claims

1. Stent (1), in particular a coronary stent, consisting of at least one thin-walled, tubular element of which the circumferential surface is openly reticular and comprises recesses (3) delimited by narrow web-like elements (4), the web-like elements in particular forming the remainder of a tubular wall from which the material has been removed in the region of the recesses, the cross-section of a web element tapering in the vicinity of the connecting region (5) with a further web element in a region of its longitudinal direction such that when the web-like element is deflected as a result of an application of force in the region of its end remote from the connecting region (11, 12), a plastic deformation over the entire web cross-section occurs in the tapered region, **characterised in that** stops (23, 24) are provided to limit the plastic deformation before a reduction in rigidity occurs owing to tears or the like, the stops being formed by extensions of the webs, outside of weakened regions, and/or regions adjacent thereto during deformation, which regions approach one another in the event of deformation on expansion of the stent and substantially prevent further deformation of the weakened region (21, 22).

2. Stent according to claim 1, **characterised in that** the deformation occurs once the flowing state of the material has been attained.

3. Stent according to claim 1, **characterised in that** the tapered region is designed such that a deformation occurs successively in local, mutually adjoining regions in the direction of increasing distance from the site of connection to the other web element.

4. Stent according to claim 1, **characterised in that** the tapered region or a region adjacent to the tapered region is designed such that in the event of material hardening in a first region located closer to the site of connection to the other web element, occurring after plastic deformation, the region of deformation is displaced into an adjacent region more remote from the site of connection than the region of hardening.

5. Stent according to claim 1, **characterised in that** the resistance to deformation increases less markedly than the material hardening increasing after the flowing state occurring owing to material deformation.

6. Stent according to claim 1, **characterised in that** the tapered region is formed by varying the web width with constant material thickness in the tangential direction and/or by removing material in the radial direction.

7. Stent according to claim 1, **characterised in that** the tapered region is provided in web regions, which in the unexpanded state of the stent adopt a position inclined to the longitudinal direction within the hollow cylindrical surface.

8. Stent according to claim 1, **characterised in that** the edges of web-like elements have rounded portions in a region of intersection or branching or in the region of a connecting region (5) connecting successive stent segments.

9. Stent according to claim 1, **characterised in that** further weakened regions are connected mechanically downstream of the stops, the weakened regions allowing further deformation on the further expansion of the stent, when the deformation of the first weakened region is substantially prevented by the stops.

10. Stent according to clam 1, **characterised in that** further stops are provided delimiting the approach of adjacent web-like elements during compression or crimping.

11. Stent according to claim 1, **characterised in that** a plurality of successive segments (2) arranged in rows in the axial direction are provided and are each connected by a web-like element provided with a tapered region.

12. Stent according to claim 1, **characterised in that** the position of the connecting region (6, 6') between a plurality of segments (2) changes from segment to segment in the tangential direction.

13. Stent according to claim 1, **characterised in that**, outside of the weakened regions, deformation regions are provided in which, on expansion of the stent, at most deformations occur in which there are no deformations impairing the rigidity of the structure.

14. Stent according to claim 1, **characterised in that** the stent consists of titanium, tantalum or another biocompatible metal or a corresponding metal alloy as material.

15. Stent according to claim 1, **characterised in that** a coating of amorphous silicon carbide is provided.

## Revendications

1. Extenseur (1), en particulier extenseur coronarien, consistant en au moins un élément tubulaire à parois minces dont la surface d'enveloppe est agencée en forme de réseau interrompu et comporte des évidements (3) qui sont limités par des éléments de type traverse (4) de faible largeur, où les éléments du type traverse forment en particulier le reste d'une paroi tubulaire, de laquelle le matériau a été retiré dans le domaine des évidements, où la section droite d'un élément de traverse à proximité du domaine de liaison (5) avec un autre élément de traverse est rétrécie dans un domaine de sa direction longitudinale de telle manière que, quand il se produit une déviation de l'élément de type traverse sous l'action d'une force dans le domaine de son extrémité distante du domaine de liaison (11, 12), il se produit dans le domaine rétréci une déformation plastique sur toute la section droite de la traverse, **caractérisé en ce qu'**il est prévu des butées (23, 24) pour limiter la déformation plastique avant qu'il se produise une diminution de la solidité par fissuration ou analogue, où les butées sont formées par des élargissements des traverses, à l'extérieur de domaines d'affaiblissement, et/ou de domaines voisins de ceux-ci lors d'une déformation, qui se rapprochent les uns des autres lors d'une déformation avec l'expansion de l'extenseur et qui empêchent sensiblement une déformation supplémentaire du domaine d'affaiblissement (21, 22).

2. Extenseur selon la revendication 1, **caractérisé en ce que** la déformation survient tandis que l'état de fluage du matériau est atteint.

3. Extenseur selon la revendication 1, **caractérisé en ce que** le domaine de rétrécissement est agencé de telle manière qu'il se produit une déformation dans des domaines localement voisins successivement dans la direction de l'éloignement croissant de l'endroit de la liaison avec l'autre élément de traverse.

4. Extenseur selon la revendication 1, **caractérisé en ce que** le domaine de rétrécissement ou un domaine voisin du domaine de rétrécissement est agencé de telle manière que le domaine de la déformation, dans le cas de la survenue d'une consolidation du matériau après une déformation plastique dans un premier domaine, qui est plus proche de l'endroit de la liaison avec un autre élément de traverse, est déplacé dans un domaine voisin qui est plus éloigné de l'endroit de la liaison que le domaine de la consolidation.

5. Extenseur selon la revendication 1, **caractérisé en ce que** la résistance à la déformation augmente moins fortement que la consolidation du matériau qui augmente après l'état de fluage qui survient par déformation du matériau.

6. Extenseur selon la revendication 1, **caractérisé en ce que** le domaine de rétrécissement est formé par variation de la largeur de la traverse pour une épaisseur de matériau constante en direction tangentielle et/ou par retrait de matériau en direction radiale.

7. Extenseur selon la revendication 1, **caractérisé en ce que** le domaine de rétrécissement est prévu dans des domaines de la traverse qui revêtent, à l'état non expansé de l'extenseur, une position inclinée par rapport à la direction longitudinale à l'intérieur de la surface cylindrique creuse.

8. Extenseur selon la revendication 1, **caractérisé en ce que** les bords d'éléments de type traverse comportent des arrondis dans un domaine de croisement ou de ramification ou dans le domaine d'un domaine de liaison (5) reliant les éléments d'extenseur successifs.

9. Extenseur selon la revendication 1, **caractérisé en ce que** d'autres domaines d'affaiblissement, qui permettent une déformation supplémentaire lors d'une expansion supplémentaire de l'extenseur, quand la déformation du premier domaine d'affaiblissement est sensiblement empêchée par les butées, font suite mécaniquement aux butées.

10. Extenseur selon la revendication 1, **caractérisé en ce qu'**il est prévu d'autres butées qui limitent le rapprochement d'éléments de type traverse voisins lors d'une compression ou d'un rétrécissement.

11. Extenseur selon la revendication 1, **caractérisé en ce qu'**il est prévu plusieurs segments (2) qui se succèdent en forme de série en direction axiale, qui sont reliés à chaque fois par un élément de type traverse qui est muni d'un domaine de rétrécissement.

12. Extenseur selon la revendication 1, **caractérisé en ce que** la position du domaine de liaison (6, 6') entre plusieurs segments (2) varie dans la direction tangentielle d'un segment à un autre segment.

13. Extenseur selon la revendication 1, **caractérisé en ce qu'**il est prévu, à l'extérieur des domaines d'affaiblissement, des domaines de déformation dans lesquels se produisent, lors de l'expansion de l'extenseur, au plus des déformations dans lesquelles il n'apparaît aucune déformation nuisant à la solidité de la structure.

14. Extenseur selon la revendication 1, **caractérisé en ce que** l'extenseur consiste, comme matériau, en titane, en tantale ou en un autre métal biocompatible ou en un alliage métallique correspondant.

15. Extenseur selon la revendication 1, **caractérisé en ce qu'**il est prévu un revêtement en carbure de silicium amorphe.
